# EUROPEAN PATENT APPLICATION

(11) **EP 1 913 872 A1**
(43) Date of publication of application: **23.04.2008**
(21) Application number: 06782500.0
(22) Date of filing: 08.08.2006
(51) Int. Cl.: A61B 5/151

(54) **NEEDLE INSERTION DEVICE, AND LANCET ASSEMBLY AND INJECTOR ASSEMBLY THAT FORM THE SAME**

(30) Priority: 10.08.2005 JP 2005231649
(71) Applicant: Izumi-Cosmo Company Limited, Osaka-shi, Osaka 530-0005 (JP)
(72) Inventor: KITAMURA, Yoritaka, Tokyo 1030022 (JP); ABE, Teruyuki, Tokyo 1030022 (JP); SEKI, Kazuharu, Tokyo 1540001 (JP)
(74) Representative: HOFFMANN EITLE
(86) International application number: PCT/JP2006/315674
(87) International publication number: WO 2007/018215

(57) **Abstract**

A pricking device is provided that allows a protruding tip of a distal end portion of a pricking member to be shielded from surround as much as possible and removed from an injector assembly.

The lancet assembly 100 contains a lancet 101 and a protective cover 102, **characterized in that**
(1) the lancet comprises a lancet body 104, lancet cap 106 and a pricking member 105, the pricking member is disposed in the lancet body and the lancet cap while straddling them members, and the distal end portion 124 of the pricking member is enclosed by the lancet cap; and
(2) the protective cover is disposed around the lancet body, so that it is able to move to a position, after pricking, around the distal end portion of the pricking member that protrudes forward from the lancet body.

## Description

### BACKGROUND OF THE INVENTION

### Field of the Invention

The present invention relates to a pricking device used in pricking a predetermined portion of a human body with a sharp pricking member such as a needle for sampling a body fluid such as blood, a lancet assembly and an injector assembly that constitute the pricking device.

### Description of the Related Art

Various pricking devices have been used to collect a small amount of blood sample for the purpose of measuring a blood sugar level of patients with diabetes. Such a device is composed of a lancet and an injector. The lancet having a pricking member that pricks a predetermined portion of the body of a patient is incorporated in the injector that ejects (or launches) a lancet toward the predetermined portion. The lancet is ejected toward the predetermined portion by making use of an expanding action of a compressed spring provided in the injector.

When taking a blood sample by using such a pricking device as described above, particular attention must be paid upon the handling of the lancet that has been used. In the lancet that has been used for pricking, typically its distal end portion of the pricking member that bears a trace amount of the patient's blood is exposed from a lancet body. Should a part of the body of a person other than the patient (for example, a nurse who collects the blood sample) accidentally touches the distal end portion of the pricking member, the body part may be pricked by the distal end portion of the pricking member causing a cut through which the patient's blood may enter the other person's body, thus posing the danger of infection of a disease.

Known pricking devices are not necessarily designed with due consideration given to the handling of the lancet that has been used. For example, it has been proposed to apply a cap on the exposed distal end portion of the pricking member after pricking (refer to Patent Reference 1 described hereinafter). This device requires to be handled in order to apply the cap thereon while the lancet is in the state in which the distal end thereof is exposed, and therefore the danger described above is not eliminated.

Accordingly, the pricking device requires utmost attention upon handling the lancet after it has been used, and there is a demand for a pricking device that allows the lancet to be handled after the lancet has been used in a safer state.
Patent Reference 1: U.S. Patent No. 5,385,571

### DISCLOSURE OF THE INVENTION

### Problem to be Solved by the Invention

An object of the present invention is to provide a pricking device of which distal end portion of the pricking member can be removed from the injector after the protruding distal end portion of the pricking member has been shielded from its surrounding as much as possible, rather than removing the pricking member from the injector after pricking while the distal end portion remains in the state in which the distal end portion protrudes from the lancet body.

### Means to Solve the Problem

In a first aspect, the present invention provides a pricking device comprising:
(a) a lancet assembly comprising a lancet and a protective cover; and
(b) an injector that has an injector body and a plunger and a pusher which are disposed in the injector body and which ejects the lancet,
characterized in that
(1) the lancet comprises a lancet body, a lancet cap and a pricking member, the pricking member is disposed in the lancet body and the lancet cap while straddling those members, and a distal end portion of the pricking member is enclosed by the lancet cap;
(2) the protective cover is disposed around the lancet body, and it is able to move to a position, after pricking, around the distal end portion of the pricking member that protrudes forward from the lancet body;
(3) the plunger holds a rear end portion of the lancet body and ejects the lancet body so that the protruding distal end portion of the pricking member pricks a predetermined portion; and
(4) the pusher pushes, after pricking, the protective cover that is disposed around the lancet body so as to move it forward, and thereby the protective cover is located around the distal end portion of the pricking member that protrudes forward from the lancet body.

In a second aspect, the present invention provides a lancet assembly comprising a lancet and a protective cover,
characterized in that
(1) the lancet comprises a lancet body, a lancet cap and a pricking member, the pricking member is disposed in the lancet body and the lancet cap while straddling those members, and a distal end portion of the pricking member is enclosed by the lancet cap; and
(2) the protective cover is disposed around the lancet body, and it is able to move to a position, after pricking, around the distal end portion of the pricking member that protrudes forward from the lancet body.

In a third aspect, the present invention provides an injector assembly comprising an injector body and a plunger and a pusher which are disposed in the injector body and which ejects the lancet that has a protective cover,
characterized in that
(1) the plunger holds a rear end portion of the lancet body and ejects the lancet body so that a protruding distal end portion of the pricking member pricks a predetermined portion; and
(2) the pusher pushes, after pricking, the protective cover that is disposed around the lancet body so as to move it forward so that the protective cover is located around the distal end portion of the pricking member that protrudes forward from the lancet body

The present invention will be explained below, in which directions meant by the terms "forward" and "backward" are used based on the direction along which the lancet is ejected, namely the direction in which the lancet moves. Specifically, the direction of the lancet movement to carry out pricking (namely the direction along which the exposed distal end portion of the pricking member moves toward the predetermined pricking point) is meant by the term "front", "forward" or "forward direction", and the direction just opposite to such direction is meant by the term "back", "backward" or "back direction". The terms "up (upward)" and "down (downward)" are used to mean the directions perpendicular to the forward and backward directions and based on the drawing(s) to which reference is made.

The pricking device of the present invention makes it possible to handle the lancet in a safer state when removing the lancet from the injector assembly after pricking, since the distal end portion of the pricking member that protrudes from the lancet body is enclosed by the protective cover.

### Brief Description of the Drawings

Fig. 1 is a perspective view of a lancet assembly that can be used in the pricking device of the present invention.
Fig. 2 is a perspective view schematically showing the lancet assembly of Fig. 1 with a half thereof on the near side being cut away.
Fig. 3 is a perspective view schematically showing a lancet 101 that constitutes the lancet assembly of the present invention.
Fig. 4 is a perspective view schematically showing a protective cover 102 that constitutes the lancet assembly of the present invention.
Fig. 5 is a perspective view schematically showing the lancet assembly with the front end portion of the pricking member to be ejected being exposed therefrom upon pricking.
Fig. 6 is a perspective view schematically showing the lancet assembly with the protective cover having moved forward after pricking.
Fig. 7 is a perspective view schematically showing the lancet assembly of Fig.6 with a half thereof on the near side being cut away.
Fig. 8 is a top view schematically showing a lancet of another aspect of the present invention.
Fig. 9 is a perspective view schematically showing the exterior of an injector assembly that can be used in the pricking device of the present invention.
Fig. 10 is a perspective view schematically showing the injector assembly shown in Fig. 9 with a half thereof on the near side being cut away to show the inside of the injector assembly.
Fig. 11 is a perspective view schematically showing a plunger 204.
Fig. 12 is a perspective view schematically showing a pusher 206.
Fig. 13 is a perspective view schematically showing a lower half 224 that constitutes the plunger 204.
Fig. 14 is a perspective view schematically showing, similarly to Fig. 10, the state in which the lancet assembly is inserted in the injector assembly with the rear end portion of the lancet body located between arms of the plunger.
Fig. 15 is a perspective view schematically showing, similarly to Fig. 10, the state in which the rear end portion of the lancet body including the protruding portion thereof is completely held between the arms of the plunger.
Fig. 16 is a perspective view schematically showing, similarly to Fig.10, the state in which the plunger is moved from the position of Fig. 15 backward so that a protrusion of the plunger abuts against a rear end portion of the trigger member, namely the state in which charging of the lancet assembly is completed.
Fig. 17 is a perspective view schematically showing, similarly to Fig. 10, the state in which the lancet cap is twisted and removed from the lancet assembly, so as to be ready for ejecting the lancet.
Fig. 18 is a perspective view schematically showing, similarly to Fig. 10, a state in which a push button has been pressed to eject the lancet, so that the distal end portion of the pricking member has protruded from an opening of the injector assembly, namely the predetermined point is pricked.
Fig. 19 is a perspective view schematically showing, similarly to Fig. 10, a state in which the lancet has retracted after completion of pricking.
Fig. 20 is a perspective view schematically showing, similarly to Fig. 10, a state in which a push bar is urged forward so as to move the protective cover forward with respect to the lancet body that has finished pricking, thus resulting in a cantilever of the plunger deflected toward the outside with the front end portion thereof protruding toward the inner wall of the injector body.
Fig. 21 is a perspective view schematically showing, similarly to Fig. 10, a state in which an end portion of the cantilever that has protruded is abutting against a stopper provided on an inner wall of the injector body by urging the push bar further forward from the state shown in Fig. 20.
Fig. 22 is a perspective view schematically showing, similarly to Fig. 10, a state in which the protective cover is caused to move forward by pushing the push bar further forward from the state shown in Fig. 21, so that the protective cover is disposed around the exposed distal end portion of the pricking member.
Fig. 23 is a perspective view schematically showing, similarly to Fig. 10, a state in which the end of the protruding cantilever is caused to restore its original form by pushing the push bar further forward from the state shown in Fig. 22, so that the plunger is enabled to move forward, and then front end portions of the plunger arms fit into recesses formed in the inner wall of the injector body, with the arms being splayed out toward the outside and a rear end portion of the lancet body being released.
Fig. 24 is a perspective view schematically showing, similarly to Fig. 10, a state in which the released lancet is moved forward from the state shown in Fig. 23.
Fig. 25 is a perspective view schematically showing, similarly to Fig. 10, a state in which the released lancet is moved further forward from the state shown in Fig. 24, so that the lancet pops out of the injector assembly.

### Description of Reference Numerals

- 100:: Lancet assembly
- 101:: Lancet
- 102:: Protective cover
- 104:: Lancet body
- 105:: Pricking member
- 106:: Lancet cap
- 108:: Weakened portion
- 110:: Tab element
- 112:: Abutment element
- 114:: Front portion
- 116:: Rear portion
- 118:: Protruding portion
- 120:: Protruding portion
- 122:: Protruding portion
- 124:: End portion of pricking member
- 125:: Opening
- 126:: Tip of end portion of pricking member
- 128:: Wall portion of protective cover
- 130:: Tapered surface
- 131:: Protruding portion
- 132:: Steep surface
- 140:: Sloped surface
- 142:: Sloped surface
- 150:: Rear end portion
- 152:: Protruding portion
- 200:: Injector assembly
- 202:: Injector body
- 204:: Plunger
- 206:: Pusher
- 208:: Cap assembly
- 210, 210':: Body half
- 212:: Cocking member
- 213:: Push bar
- 214:: Opening
- 220:: Main body
- 222:: Upper half
- 224:: Lower half
- 226:: Rear portion
- 228:: Upper half
- 230:: Lower half
- 232:: Cantilever
- 234:: Rear end
- 235:: Sloped surface
- 236:: Front end
- 237:: Protrusion
- 238:: Inside surface
- 239:: Recess in front end portion
- 240:: Inside surface
- 241:: Recess in front end portion
- 242:: Recess (or pedestal)
- 243:: Protrusion
- 244:: Rod-like portion
- 245:: Recess
- 246:: Arm
- 247:: Bottom of recess
- 248:: Arm
- 249:: Sloped surface
- 250:: Front portion
- 252, 253, 254, 256:: Portion of pusher
- 260:: Space in rear portion
- 262:: Recess
- 264, 266:: Front end portion of arm
- 268, 270:: Curved surface
- 502, 504:: Outermost portion of arm distal end portion
- 506, 508:: Recess
- 510, 512:: Innermost portion of arm distal end portion
- 514:: Trigger member
- 516:: Fulcrum
- 518:: plate
- 520:: Spring
- 524:: Protrusion
- 526:: Rear end portion
- 530:: Rear end portion of main body
- 532:: Front end portion of cocking member
- 534:: Partition member
- 536:: Rear end portion of cocking member
- 538:: indicator
- 540:: Opening
- 542:: Push button (front end portion)
- 550:: Protrusion
- 560, 562:: Recess in inner wall of injector body
- S1:: Injection spring
- S2:: Return spring
- S3:: Pushback spring

### Embodiments to Carry Out the Invention

The pricking device of the present invention will now be described with reference to the accompanying drawings. It is noted that in the lancet assembly, the injector assembly and the pricking device according to the present invention, a pricking member is formed from a metal (for example, a stainless steel), a spring that produces energy for moving the lancet before and after pricking (including "during" pricking) may be formed of any appropriate material such as a plastic, preferably a metal, and other parts are preferably, and are usually, formed of a proper plastic material(s). In the description below, it is assumed that various members and elements are formed of such materials as described above, unless otherwise noted. For example, the pricking member is formed of a metal (for example, a metal needle), the spring is also formed of a metal (for example, a coil spring) and the other members are formed of an appropriate plastic material(s) (for example, molded members). The plastic material is formed into a predetermined structure typically by injection molding. Plastic materials that can be used are, for example, a low-density polyethylene resin (LDPE), a high-density polyethylene resin (HDPE), a polypropylene resin (PP) and the like for the lancet, and a polycarbonate resin (PC), an acrylonitrile-butadiene-styrene copolymer resin (ABS), a polyacetal resin (POM), a polystyrene resin (PS) and the like for the injector assembly and the protective cover.

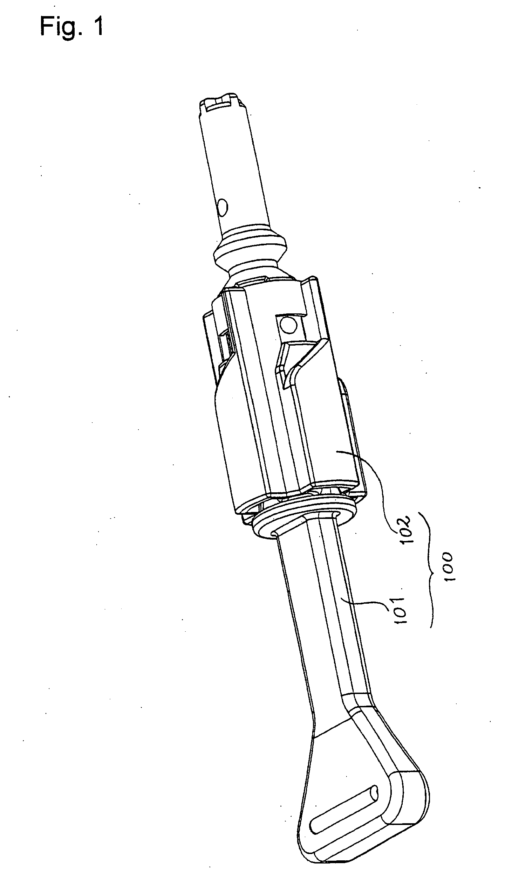

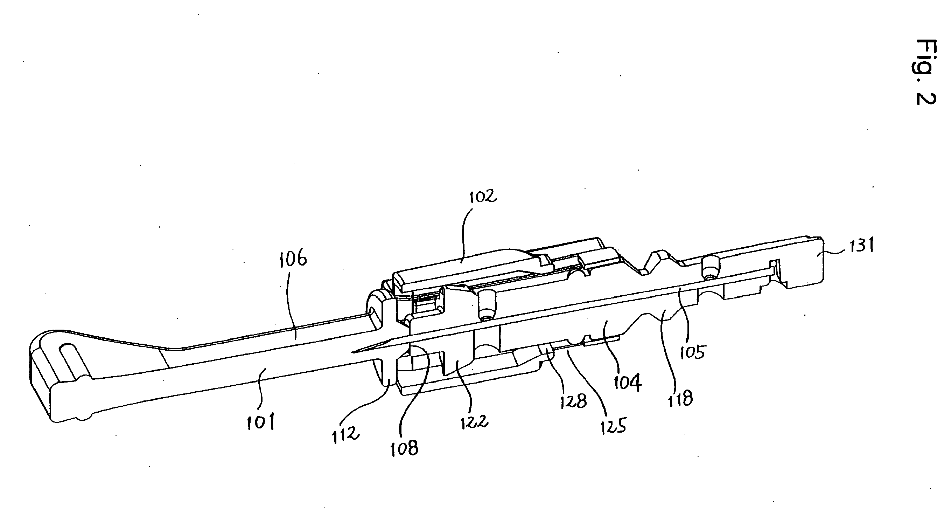

Fig. 1 shows in a perspective view the lancet assembly that can be used in the pricking device according to the present invention, and Fig. 2 shows in a schematic perspective view the lancet assembly with its half thereof on the near side being cut away. The lancet assembly 100 shown in the drawings is composed of a lancet 101 and a protective cover 102, and in the embodiment shown in the drawings, the protective cover is disposed around the lancet body 104 of the lancet 101.

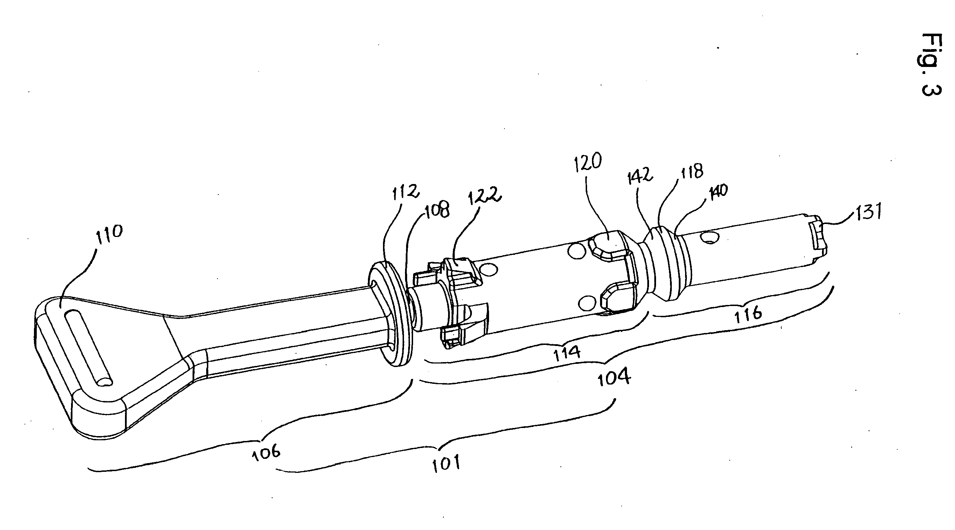

Fig. 3 shows the lancet 101 that constitutes the lancet assembly of the present invention in a schematic perspective view. The lancet 101 is composed of the lancet body 104 and the lancet cap 106. As can be seen from Fig. 2, the pricking member 105 having a tip in its end portion that tip punctures a predetermined portion is embedded in the lancet, with the distal end portion disposed in the lancet cap 106 and the other portion disposed in the lancet body 104.

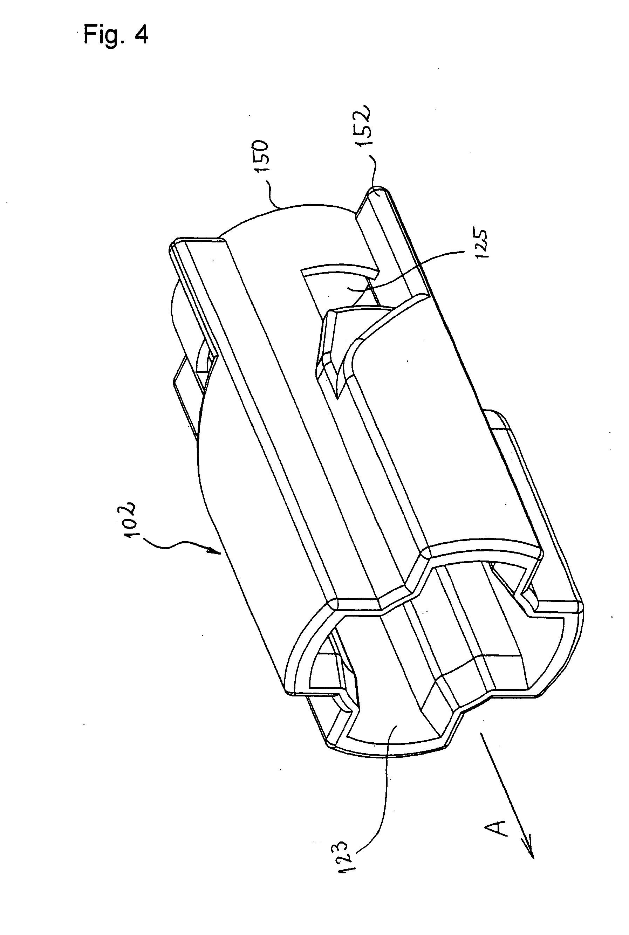

Fig. 4 shows the protective cover 102 that constitutes the lancet assembly of the present invention in a schematic perspective view. In the embodiment shown, the direction indicated by the arrow A is an ejecting direction of the lancet, namely the forward direction. As will be easily understood, it is noted that the lancet assembly of the present invention can be formed by inserting the rear portion 116 of the lancet body through an opening 123 formed in the front end portion of the protective cover 102 in a direction opposite to the arrow A.

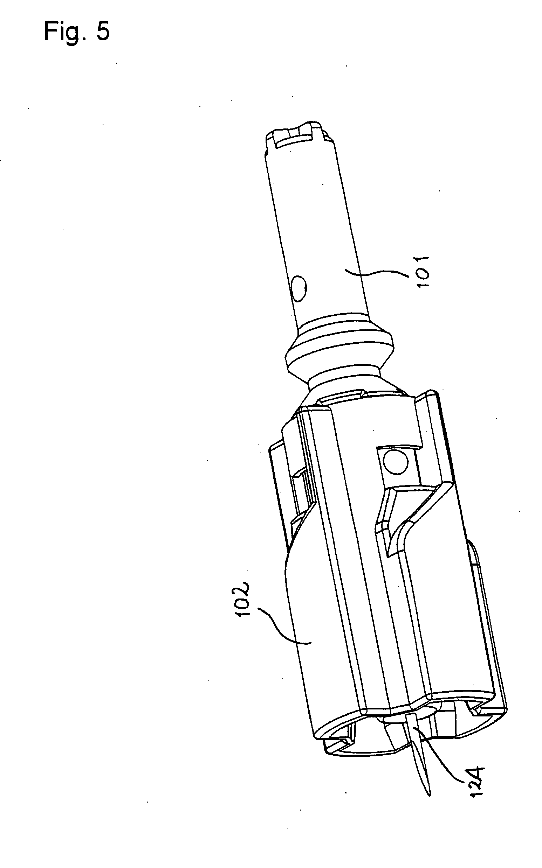

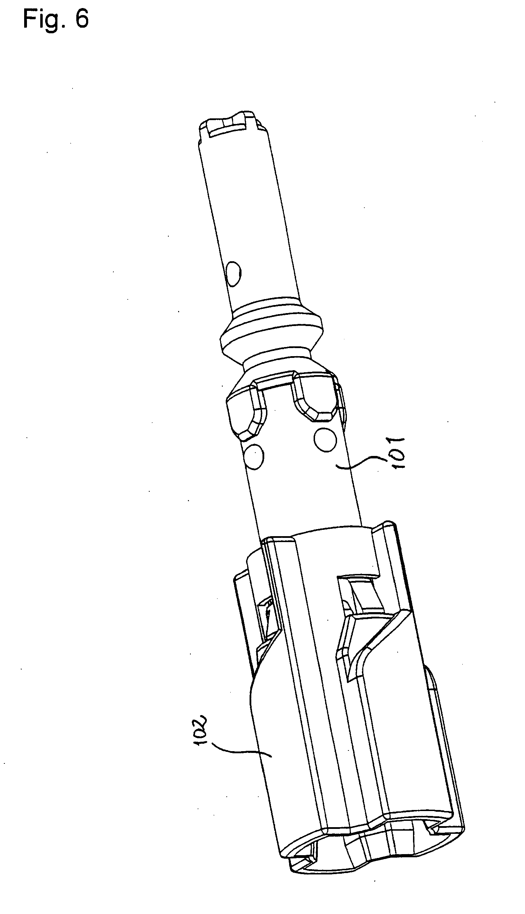

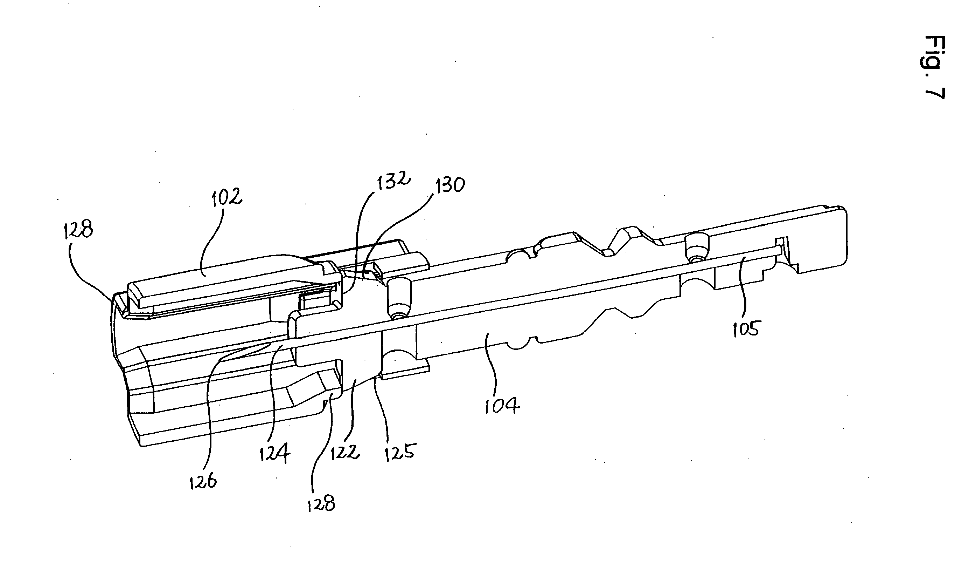

The lancet assembly composed of the lancet 101 and the protective cover 102 as described above is inserted in the injector assembly that launches the lancet and is charged in the state shown in Fig. 1. Then, after the lancet has been ejected in the state in which the distal end portion 124 of the pricking member 105 is exposed as shown in Fig. 5, the protective cover can be moved forward as shown in Fig.6 and in Fig. 7. The states shown in these drawings are different from the states shown in Fig. 1 and in Fig. 2 in that the distal end portion of the pricking member 105 is exposed and that the protective cover has moved forward, but there is no substantial difference in other respects between those states.

The lancet body 104 and the lancet cap 106 are integrally connected by a weakened portion 108 located therebetween. The weakened portion can be broken by turning the lancet body 104 and the lancet cap 106 in directions opposite to each other around the pricking member, in other words can be twisted off. The lancet having such a structure is preferably formed integrally through insertion molding of a plastic material with the pricking member 105 located in a mold in advance, and the weakened portion can be formed by decreasing the thickness of the plastic material around the pricking member.

For the purpose of making it easier to twist off, a tab element 110 is provided to the lancet cap 106, which makes it easy to turn by holding it with fingers. The tab may be, for example, a flat extending portion. In the embodiment shown, such a portion is provided in a front portion of the lancet cap 106. In the rear end portion of the lancet cap 106, an abutment element 112 is provided, which may have a form of a protrusion that extends from a lancet cap toward its outside, for example in the form of a flange.

As will be understood from Fig. 1 and Fig. 2, the abutting element 112 can function as a stopper when the lancet body 104 is inserted through the opening 123 of the front end portion of the protective cover 102 and the protective cover 102 is disposed around the lancet body 104, particularly the front portion 114 thereof, so that the front end portion of the protective cover 102 is put into contact with the abutting element 112 and thereby the protective cover 102 cannot proceed forward any more.

The rear portion 116 of the lancet body 104 can be disposed between opposing arms of the plunger of the injector assembly so that a protrusion 118 provided on the rear portion 116 is held between recesses formed on the insides of the arms, as will be described later. As the protrusion 118 is held in this way, the lancet is held by the plunger in the injector assembly.

The front portion 114 of the lancet body has protrusions 120 on its rear end thereof, and other protrusions 122 on its front end. The protrusions 120 and 122 locate the protective cover 102 with respect to the lancet 101 when the protective cover 102 is disposed around the lancet body 104. With such location, the positional relationship between the protective cover 102 and the lancet 101 is changed by a force exerted by the pusher so as to move the protective cover 102 as will be described later. That is, the protective cover 102 can be moved forward, but when such a force is not applied, the positional relationship does not substantially change.

Specifically, the protrusions 120 are disposed so as to oppose each other on the side face of the front portion 114 of the lancet body 104, and also the protrusions 122 are similarly disposed on the side surface. The distance between the opposing protrusions 120 and the distance between the opposing protrusions 122 may be substantially equal to or slightly larger than the distance between points on the inner wall of the protective cover that points correspond to the positions where the protrusions are located when arranged as shown in Fig. 1 and Fig. 2, so that the location described above is made possible in the state shown in Fig. 1 and Fig. 2 by making use of the elastic deformation of the plastic material. That is, the external profile of the protrusion 120 is substantially equal to or slightly larger than the inside profile of the protective cover, and the same is applied to the protrusion 122. Such location may be termed as press fitting of the lancet body 104 and the protective cover 102.

When the protective cover 102 moves forward to completely cover the distal end portion 124 of the pricking member that has been exposed, and then the tip of end portion 126 has been placed in the state shown in Fig. 6 and Fig. 7 that is located sufficiently backward from the front end surface of the protective cover 102, the protrusions 122 fit into the openings 125 formed on the side face in the rear of the protective cover 102. As a result, in the state shown in Fig. 6 and Fig. 7, even when such a force is exerted that causes the protective cover 102 to move back in relation to the lancet body 104, moving back is prevented by the fitting in design. In other words, the openings 125 of the protective cover 102 and the protrusions 122 of the lancet body 104 cooperate to prevent the protective cover 102 from its backward movement. Since it is necessary that a portion 128 of the wall just in front of the opening of the protective cover is capable of moving forward by riding over the protrusion 122, the protrusion 122 has a tapered surface 130 that splays out in the forward direction as shown in the drawing, and also has a surface of a steep slope with respect to the side face of the protective cover 102, preferably a substantially vertical surface 132, located adjacent to the tapered surface 130 in order to prevent the protective cover 102 from moving back.

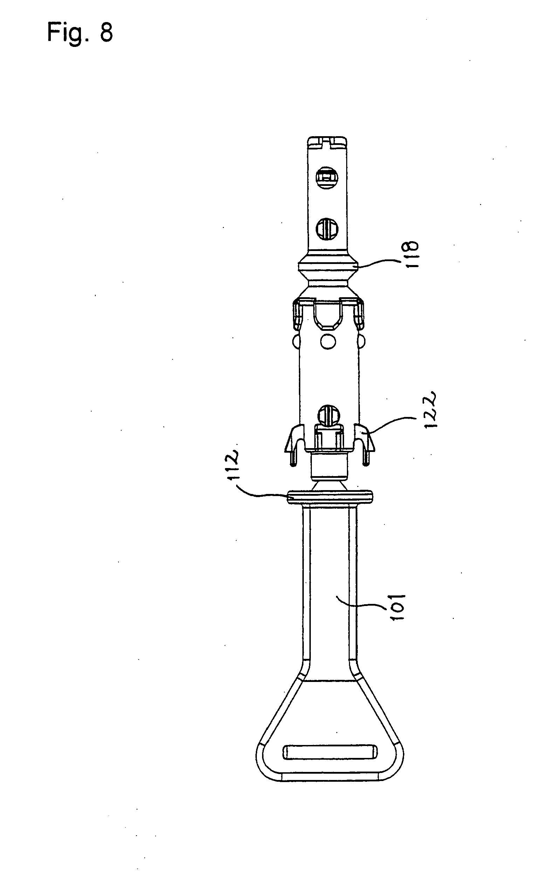

In a particularly preferable embodiment, as shown in Fig. 8, the protrusion 122 extends vertically from the side surface of the front end of the front portion 114 of the lancet body or the vicinity of such end (for example, a little backward point from the front end as shown in the drawing), and then bends to extend forward (the bend may be a curved portion), that is, protrudes from the side face of the lancet body forward 114 in a semi U-shaped form, as shown in Fig. 8. It is particularly preferable as shown in Fig. 8 that a portion of the protrusion near its base thereof has such a form that it can be tightly received by the rear end portion of the protective cover, especially by the opening thereof, or such a form that the rear end fits in and the rear end portion of the protective cover is sandwiched between the front end portion of the lancet body and the protrusion 122.

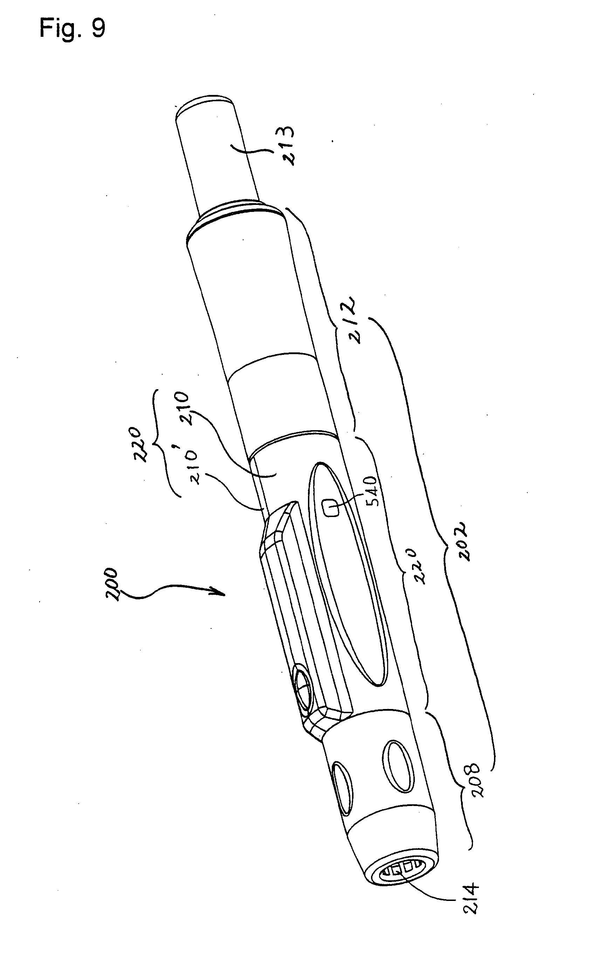

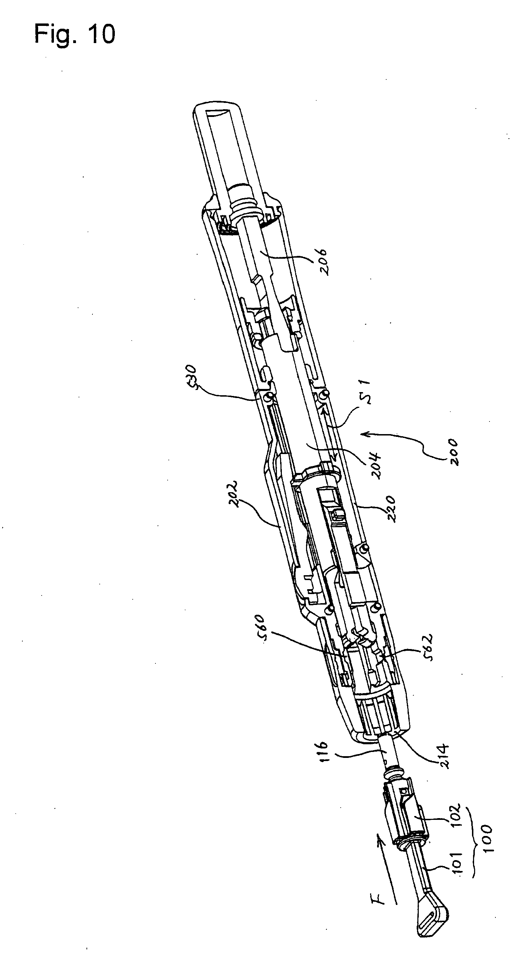

Fig. 9 shows an exterior of an injector assembly that can be used in the pricking device of the present invention, in a schematic perspective view, and Fig. 10 shows the injector assembly shown in Fig. 9 with a half thereof on the proximal side being cut away to show the inside of the injector, in a schematic perspective view. This state is a state of waiting for the lancet to be charged, namely a standby state.

The injector assembly 200 of the present invention comprises an injector body 202, and a plunger 204 and a pusher 206 which are disposed in the injector body. The injector assembly receives the lancet assembly 100 having the protective cover described above, and is capable of ejecting the lancet with the distal end portion of the pricking member exposed. The injector body 202 is composed of a cap assembly 208, a pair of body halves 210 and 210' and a cocking member 212. For the sake of simplicity, the body halves 210 and 210' put together will be referred to as a main body 220. The injector assembly 200 has, in the front end portion thereof, an opening 214 on which a predetermined portion to be pricked (for example, a finger tip) is pressed. More particularly, the cap assembly 208 defines the opening 214 in the front end portion thereof. It is noted that the cap assembly 208 preferably has a mechanism for changing the depth of pricking.

The mechanism for changing the depth of pricking is known from, for example, WO1997/004707 (which corresponds to Japanese Patent No. 3,638,958, and U.S. Patent No. 5,730,753), the disclosure of which is incorporated by reference herein. For example, a mechanism is employed in which the distance can be changed between the opening 214 and an element to which the lancet body 104 collides so as to instantaneously stop the forward movement of the lancet body 104 with the tip of the distal end portion of the pricking member exposed and then to return the lancet 104 when the lancet body is ejected (an element that is provided in the injector body and functions as a stopper). For example, such an element that functions as a stopper is provided in the front end of the body half, and a means is provided which is able to change the distance between such element and the opening 214.

For example, the distance can be changed by making an arrangement in which the cap assembly 208 can be secured by screwing in the front end portion of the main body 220. By rotating the cap assembly 208 along a screw provided around the main body 220, the position of the cap assembly 208 is changed in the longitudinal direction of the main body 220, so that the distance described above changes.

The plunger 204 holds the rear end portion 116 of the lancet body, and has a function to eject forward the lancet body 104 with the distal end portion 124 of the pricking member 105 protruding, so that the distal end portion 124 of the pricking member 105 pricks the predetermined portion. After pricking, the plunger 204 also has a function to retract backward the lancet body 104 with the distal end portion 124 of the pricking member 105 protruding.

After pricking, the pusher 206 pushes the protective cover 102, that is disposed around the lancet body 104 which has been retracted, forward with in relation to the lancet body 104, and moves the protective cover 102 to a position around the distal end portion 124 of the pricking member 105 that protrudes forward from the lancet body 104.

In the injector body 202 of the present invention, the cap assembly 208 is disposed around the front end portion of the pair of body halves 210 and 210' that are assembled together (for example, by snap fit) and define an inner space therein. The inner space thus defined accommodates the plunger 204 and the pusher 208 disposed therein, as described above.

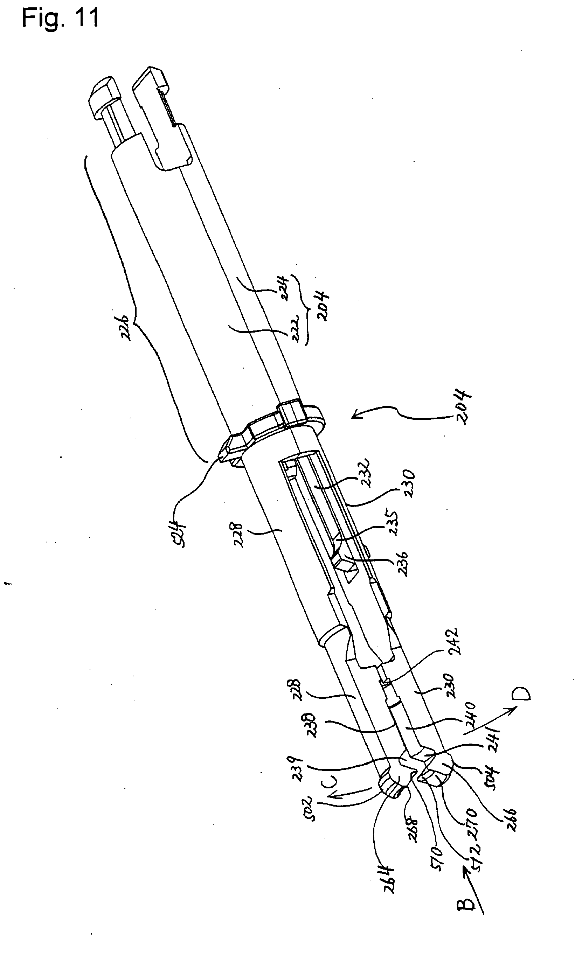

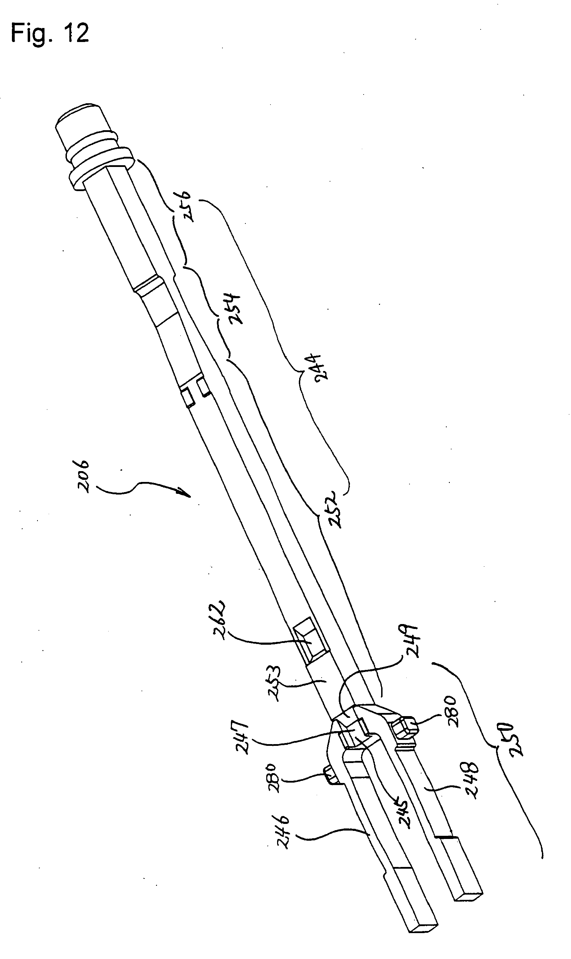

Fig. 11 shows the plunger 204 in a perspective view, and Fig. 12 shows the pusher 206 in a schematic perspective view. The plunger 204 comprises an upper half 222 and the lower half 224, which are shown as being combined together (for example, by snap fit).

The plunger 204 comprises two opposing arms of an upper arm 228 and a lower arm 230 that extend forward from a front end of a cylindrical rear portion 226, that define a space passing therethrough. The space defined within the rear portion does not necessarily have a circular cylindrical form, and it is in a rectangular cylindrical form in the shown embodiment. The halves 222 and 224 that define the plunger 204 have substantially the same shape, but they are different in that the lower half 224 has a cantilever 232 and the upper half has a protrusion 524.

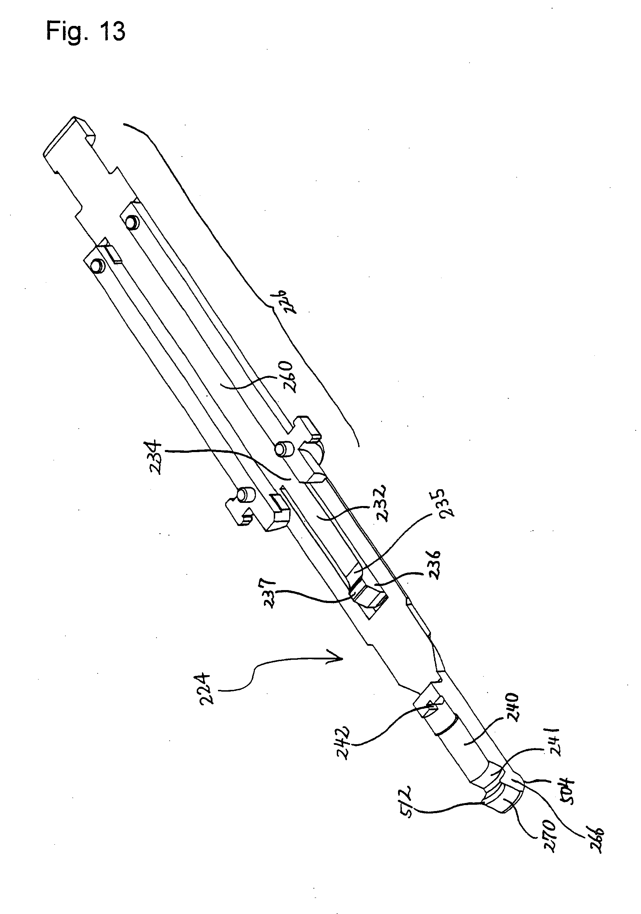

Fig. 13 shows the lower half 224 that constitutes the plunger 204 in a schematic perspective view. The cantilever 232 has a form of a beam supported only at one end, seen from the meaning of "cantilever", with its rear end 234 being connected integrally to the vicinity of the front end portion of the rear portion of the lower half 224, while its front end 236 is free and has a protrusion 237 provided thereon. The protrusion has a rear sloped surface 235 that defines the protrusion. The plunger 204 is preferably formed of a plastic material as a whole. Therefore, when the cantilever 232 receives a force acting downward on the front end 236, the protrusion 237 or in the vicinity of the cantilever, the front end 236 deflects downward so that the entire cantilever elastically deforms into a curved form. On the contrary, when the force is removed, the original shape shown in Fig. 13 is restored.

The upper arm 228 and the lower arm 230 of the plunger 204 have recesses 239 and 241, respectively, in the front end portions thereof. A part of the protrusion 118 provided on the rear end portion 116 of the lancet body 104 fits between these recesses. Thus such arms, particularly the recess 239 and the recess 241 thereof, constitute a chuck element. These recesses preferably have such a form in which a part of the protrusion 118 just fits. Specifically, it is preferable that the recess 239 and the recess 241 have complementary shapes with the protrusion 118 with respect to a cross section along the longitudinal direction of the pricking member.

Inside surfaces 238 and 240 that oppose to each other at positions behind those recesses of the arms preferably correspond to parts of the side surface of the rear portion 116 of the lancet body 104. That is, the inside surfaces 238 and 240 are preferably complementary with the side surfaces of the rear portion 116 of the lancet body 104. Such a configuration makes it possible not only to hold, between the arms, not only the protrusion 118 but also the other portion of the rear portion 116 that has no protrusion as long as the arms 228 and 230 do not splay, but also to prevent the axis of the lancet body from undergoing substantial deviation with respect to the plunger even when some force is exerted on the rear portion, so that these inside surfaces act as a guide for backward movement of the rear portion 116. Accordingly, it is preferable that the chuck element described above further comprises the inside surfaces which are located behind the recesses and complementary with the rear end portion of the lancet body. In one embodiment, there is a pedestal at a position ahead of the plunger, preferably behind the inside surface described above. In a preferred embodiment, the inside surfaces 238 and 240 constitute the chuck element, and the pedestal is located at the deepest position of the space that is formed between the inside surfaces 238 and 240.

In the embodiment shown, recesses 242 (refer to Fig. 13) are formed behind the inside surfaces 238 and 240. The recesses 242 are designed to receive at least a part of the protrusion 131 that is provided on the rear end portion of the lancet body 104 so as to just fit therein. In other words, these recesses 242 play the role of the pedestal (or seat) that receives the rear end portion of the lancet body. In the embodiment shown, a cross-shaped protrusion 131 is provided on the rear end surface of the lancet body 104, and' a part thereof fits into the recesses 242. When the plunger arm and the lancet body are provided on their ends with the means (for example, a recess and a protrusion) that engage with each other formed, the lancet is prevented from rotating relative to the plunger around the pricking member.

When charging the lancet assembly, the rear end portion 116 of the lancet body 104 is inserted through the opening 214 of the injector assembly 200 into its inside as indicated by the arrow F in Fig. 10 or in the direction indicated by the arrow B in Fig. 11, and moves toward the plunger 204 that is disposed in the injector assembly. At this time, the rear end portion of the lancet body 116 that is inserted passes between the front end 264 of the arm 228 and the front end 266 of the arm 230, and a part of the rear end portion 116 of the lancet body 116 enters between the inside surfaces 238 and 240 and the protrusion 118 of the lancet body 116 abuts against the distal end portions 264 and 266. As will be readily understood, the plunger may have two opposing arms, preferably arms of a U-shape as a whole as described above. In other embodiment, more than two arms may be provided as long as the rear end portion of the lancet body can be properly held. Therefore, the plunger has at least two arms.

The protrusion 118 may have the form of, for example, a rim or flange, that protrudes from the side face of the lancet body 104. In one embodiment, as shown in Fig. 3, the protrusion 108 is defined by sloped surface 140 (corresponds to a side surface of a truncated cone in the embodiment shown) and sloped surface 142 that extend from the side surface of the lancet body 104 toward the outermost profile of the protrusion. The distal end portion 264 and the distal end portion 266 of the arms 228 and 230, on the other hand, have curved surfaces 268 and 270 that splay out in the ejecting direction of the lancet as shown in Fig. 11, and the curved surfaces are complementary with the sloped surface 140.

As a result, when the lancet body 104 is inserted as described above and the protrusion 118 abuts against the distal end portion 264 and the distal end portion 266 of the arms, the sloped surface 140 that splays out in the ejecting direction of the lancet pushes the sloped surfaces 268 and 270 that splay out in the ejecting direction of the lancet, so that forces are exerted to move the distal end portions 264 and 266 of the arms of the plunger upward and downward, respectively.

The arm 228 and the arm 230 are combined integrally with the rear portion 226 of the plunger 204 in the rear end portion thereof. Therefore, when the forces described above are exerted, the distal end portions 264 and 266 of the arms are urged to move in the directions indicated by the arrow C and the arrow D so that the arm 228 and the arm 230 undergo elastic deflection in a small amount (namely, splay outwardly). As a result, the spacing between the distal end portions 264 and 266 increases so that the protrusion 118 of the lancet body 104 fits into the recess 239 and the recess 241 located immediately behind the end portion of the arms while passing between the distal end portions of the arms with being assisted by guiding of the backward movement of the rear end portion 116 of the lancet body 104 by means of the inner surfaces 238 and 240.

After the protrusion 118 has fitted in the recesses 239 and 241, since the recesses have complementary shapes with the protrusion, the forces acting on the arms 228 and 230 substantially disappear. As a result, the elastically deflected arms restore their original forms so as to complete the lancet grasp by the arm 228 and the arm 230. In order to enable the arms to undergo the temporary elastic deflection as described above in the injector assembly, and also after the grasp of the lancet, to prevent the arms from undergoing elastic deformation, there are such recesses on the injector body, specifically on an inner wall of the main body of the injector body, that are shaped to correspond to the shapes of the upper side of the distal end portion 264 of the arm and the lower side of the distal end portion 266 of the arm, respectively. Further, in a region where no such recesses exist, the distal ends of the arms cannot move upward or downward. On the other hand, in a region where such recesses exist, the shaped portion of the upper side and the shaped portion of the lower side of the distal end portions respectively fit into the recesses, so that the distal end portion can move upward or downward (namely splay toward the inner wall of the injector body), which makes it possible to enable the elastic deflection.

The plunger 204 as described above is used in combination with the pusher 206 as shown in Fig. 12. The pusher 206 comprises a rod-like portion 244, and a front portion 250 composed of an arm 246 and an arm 248 that extend forward from the front end of the rod-like portion. The arm 246 and the arm 248 form a U-letter shape as a whole, with a recess 245 formed near the base of the arms, and a sloped surface 249 is formed that connects the bottom 247 of the recess and the side surface of the rod-like portion 244.

The pusher 206 shown in the drawing is inverted upside down and is then disposed between the upper half 222 and the lower half 224 of the plunger shown in Fig. 11. Then the halves are combined to form the plunger 204, thereby put into operable in combination with the plunger 204. The rod-like portion 244 that is adjacent to the front portion 250 of the pusher 206 has a rectangular cross section that remains constant over a relatively long length from its front end portion toward its rearward (portion 252), and then the thickness of the rectangular section gradually decreases (portion 254), before increasing again (portion 256).

The portion where the cross section is rectangular and remains constant (portion 252) is designed so as to be capable of moving, preferably sliding, back and forth within the space 260 defined by the rear portion 226 of the plunger. When the pusher 206 moves forward in the space 260, the protrusion 237 of the front end portion 236 of the cantilever 232 provided on the lower arm of the plunger 204 fits into the recess 245 defined at the base of the arm 248 and 246. Then, as the movement continues, the sloped surface 249 that is adjacent to the recess 245 abuts against the sloped surface 235 of the plunger.

When the movement is continued further, since the sloped surface 235 and the sloped surface 249 move in directions relatively opposite to each other while the vertical movement of the portion 252 of the pusher is restricted by the portion located near the rear portion 226 of the upper arm 228 and the lower arm 230 of the plunger 204, the sloped surface 249 of the plunger 206 presses the protrusion 237 of the cantilever 232 downward. As a result, the cantilever 232 undergoes elastic deflection downward. That is, the cantilever is put into a state of being biased downward so as to press the portion 253 of the pusher upward.

When the pusher is moved further in the above described state, the recess 262 formed further behind the sloped surface 249 of the pusher 206 approaches the protrusion 237 of the plunger 204, and thereafter the protrusion 237 fits into the recess 262 because of the elasticity of the cantilever. In such a state in which the protrusion 237 has fitted in the recess, no force acts on the cantilever, so that the cantilever restores its original shape as shown in Fig. 13.

By making use of the action of the protrusion of the cantilever as described above, it is made possible for the plunger 204 and the pusher 208 to move independently from each other until pricking of the predetermined position is completed after charging the lancet assembly in the injector assembly, and also made possible for the pusher to restrict the plunger so as to prevent the movement of the plunger when the protective cover is moved forward around the lancet body after pricking. Specifically, as the end portion of the cantilever that has been elastically deflected downward abuts against the injector body, specifically the inner wall of the main body of the injector body, the plunger 204 does not move forward with respect to the injector body and remains in a fixed state due to a frictional force between the end portion of the cantilever and the inner wall, even when a force is exerted on the plunger 204 to urge forward.

In a particularly preferable embodiment, a stopper, for example a protrusion, is provided, at a position just in front of the end portion of the cantilever that has been deflected downward, on the injector body, specifically the inner wall of the main body of the injector body. Providing such a stopper is particularly effective in such a case in which the plunger cannot be sufficiently prevented from moving only by the frictional force between the end portion of the cantilever and the inner wall, and it ensures that the plunger 204 is prevented from its moving. It is preferable to provide a protrusion similar to the protrusion 237 also on a back side (namely the lower side) of the front end portion of the cantilever, which makes it easier and reliable to engage such protrusion with the stopper.

The pricking device of the present invention is constituted by loading the lancet assembly of the present invention as described above into the injector assembly of the present invention as described above. Therefore, the lancet assembly of the present invention as described above and the injector assembly of the present invention as described above in the state before such loading, namely the lancet assembly of the present invention as described above and the injector assembly of the present invention as described above that are present separately and have not yet put together constitute a kit of the pricking device.

Now by making reference to Fig. 10 and Figs. 14 to 25, steps of constituting the pricking device of the present invention by using the lancet assembly and the injector assembly of the present invention as described above, pricking a predetermined portion and then taking the used lancet out of the injector assembly will be described in order.

First, the lancet assembly 100 is charged in the injector assembly 200 that is in the standby state shown in Fig. 10. Specifically, as indicated by the arrow F in Fig. 10, the rear portion 116 of the lancet body 104 is inserted through the opening 214 of the injector assembly 200. It is preferable that the outer profile of the cross section of the protective cover which section is perpendicular to the ejecting direction is complementary, while being separated by a little gap, with a profile of a cross section of an inner wall, particularly in the vicinity of the opening 214, of the front portion of the injector body which section is perpendicular to the launching direction, to such an extent that the protective cover 102 can easily pass through the front portion of the injector body, particularly through the opening 214 thereof, but is not capable of rotating within the front portion. With such a complementary configuration, it is made easier to locate the protective cover, and therefore the lancet assembly 100 relative to the injector body, and particularly with respect to the direction around the ejecting direction.

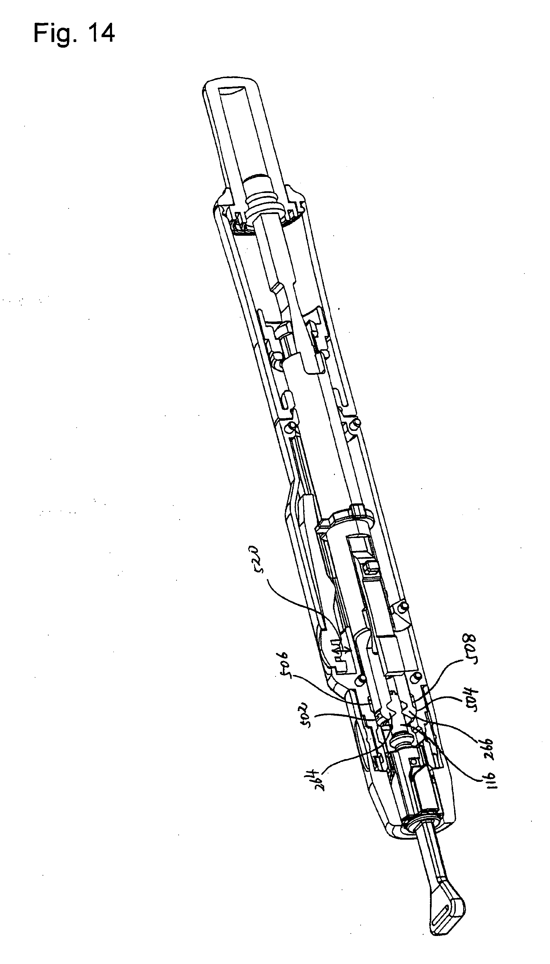

As the inserting action is continued as described above, the rear portion 116 passes between the distal end portion 264 of the plunger arm 228 and the front end portion 266 of the plunger arm 230, and then also between the recess 239 and the recess 241, sliding between the inner surfaces 238 and 249 of the arms that have complementary shapes with the side face of the rear portion 116 as shown in Fig. 14. Then, the protrusion 118 provided on the rear portion 116 abuts against the distal end portions 264 and 266 of the arms.

As described above, since the distal end portions 264 and 266 have sloped surfaces 268 and 270 that splay out in the ejecting (or launching) direction of the lancet, and the sloped surfaces have the complementary shapes with the sloped surface 140 of the protrusion 118, an attempt to move the lancet assembly further backward after the abutment causes the arms 228 and 230 to splay outward as indicated by the arrows C and D in Fig. 11. In this case, such a splaying action can be suppressed by a design such that the outermost portions 502 and 504 of the distal end portions of the arms slide over the inner surface of the injector body, specifically the inner wall of the main body of the injector body. To the contrary, in a case in which the recesses 506 and 508 are formed on the inner wall, the arms splay outward by just the depths of the recesses only when the distal end portion retracts while sliding and the outermost portions 502 and 504 thereof are fitted in the recesses 506 and 508.

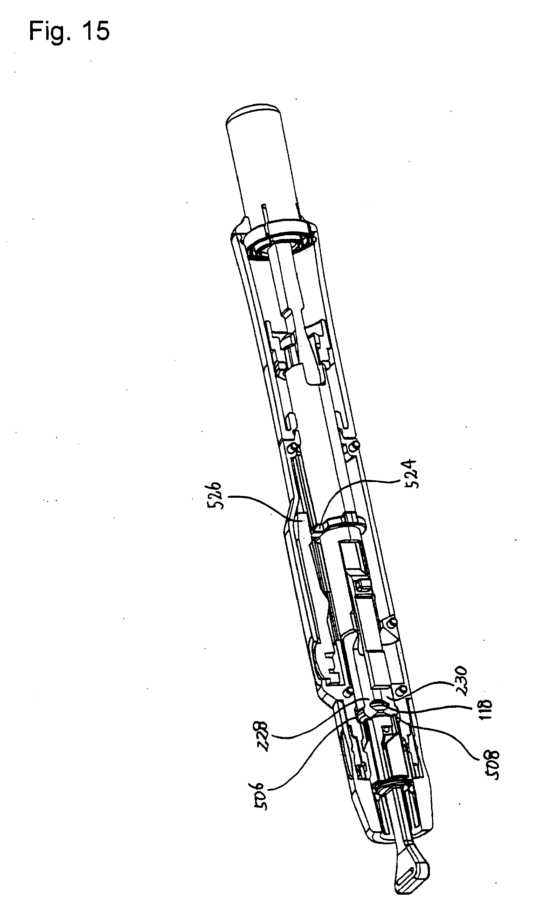

By outward splaying in this way so as to secure a distance between the innermost portions 510 and 512 of the distal end of the arms such that the protrusion 118 provided on the rear portion 116 of the lancet body can pass between those portions, the rear portion 116 is enabled to move further backward. As a result, the protrusion 118 provided on the rear portion 116 fits in the space between the recesses 239 and 241 in the distal end portions of the arms as shown in Fig. 15. When the surfaces that define the recesses 239 and 241 are formed complementary with the surfaces that define the protrusion 118, the protrusion 118 is able to fit tightly in the space between the recesses 239 and 241. Because lengths of the recesses 506 and 508 formed in the injector body, specifically on the inner wall of the main body of the injector body, are short with respect to the ejecting direction as shown in Fig. 15, a force that pushes the lancet backward applied subsequently causes the arms 228 and 230 only to retract without splaying outwardly, and therefore the lancet assembly 100 remains tightly grasped by the arms 228 and 230 of the plunger.

Then, when a force is exerted to push the lancet assembly backward, the plunger is able to move backward. In the meantime, the injector assembly 200 has a lever-like trigger member 514 that functions to trigger the ejecting of the lancet. In the embodiment shown, the trigger member 514 can move (pivot) around a portion 516 as a fulcrum, and a spring 520 (indicated by an arrow for the sake of simplicity) is provided between a front end portion 542 and a plate 518 disposed below thereof in a compressed condition. The trigger member can move like a seesaw around the fulcrum 516. While the front end portion 542 receives a force to urge upward from the spring 520, upward movement of the front end portion of the trigger member is restricted by the injector housing.

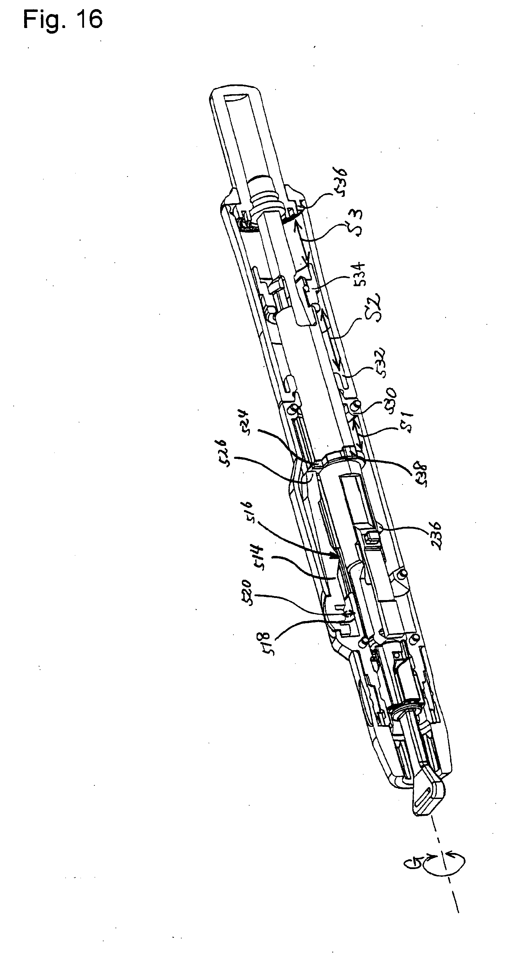

When the plunger moves further backward, the protrusion 524 that is provided integrally with the plunger moves backward together, as described above. The upper part of the protrusion 524 contacts the rear end portion 526 of the trigger member 514, and then the protrusion 524 moves back further while exerting a force directed upward on the rear portion 526 (therefore while compressing the spring 520). When the protrusion 524 passes the rear end portion 526 of the trigger member 514, such contact relationship between the trigger member 514 and the protrusion 524 is disengaged, so that the compressed spring 520 expands again and the trigger member 514 also restores its original state (the state as shown in Fig. 14). As a result, the rear end of the trigger member 514 moves down so as to prevent the protrusion 524 from moving forward. The state of the protrusion 524 having passed the rear end portion 526 and trigger member 514 having just returned to the original state in the above described way is shown in Fig. 16. This is the state in which loading, that is, charging of the lancet assembly 100 has been completed.

It is noted that inside the main body 220, a spring S1 (indicated by the arrow) is located between the protrusion 524 and the rear end portion 530 of the main body around the plunger. The spring S1 can be compressible. When the lancet assembly is inserted as described above, the spring S1 is compressed from the state shown in Fig. 10 to the state shown in Fig. 16. In the state shown in Fig. 16, the length of the spring S1 (that is, the distance between the protrusion 524 and the rear end portion 530) has become smaller than that in the state shown in Fig. 10. Since the rear end portion 526 of the trigger member 514 prevents the forward movement of the protrusion 524, namely the forward movement of the plunger 204 as described above, the spring S1 is kept in the compressed state.

When the rear end portion 526 of the trigger member 514 moves upward from this state, that is, when the front end portion 542 of the trigger member is pressed down so as to compress the spring 520, the rear end portion 526 of the trigger member 514 is displaced upward to become unable to prevent the forward movement of the protrusion 524. As a result, the spring S1 that has been kept in the compressed state as described above instantaneously expands so that the protrusion 526, therefore the plunger, and therefore the lancet held by the plunger move forward. In such a state in which the front end portion of the pricking portion is exposed, as will be described later, since the operation described above of the trigger member 514 removes what restricts the compressed spring S1, the spring S1 expands instantaneously so that the lancet is ejected to prick the predetermined portion against which the opening 214 of the injector assembly is pressed.

Within the cocking member 212, a spring S2 is disposed around the plunger between the front end portion 532 of the cocking member 212 and a partition member 534 installed on the rear portion of the plunger, and a spring S3 is disposed between the rear end portion 536 of the cocking member 212 and the partition member 534. It is noted that the cocking member 212 can slide back and forth along the inner surface of the cocking member while the partition member 534 is secured onto the rear portion of the plunger. As a result, the extents of compression of the spring S2 and the spring S3 changes in accordance to the position of the partition plate 534.

When the lancet is ejected, the rear portion of the plunger 204 moves forward and therefore the spring S2 is compressed instantaneously, but expands toward the original length immediately thereafter. Accordingly, the spring S2 functions to retract the lancet backward that has been ejected. The spring S3 has a function to retract the push bar 213 backward that has moved forward so as to exert a force to move the pusher forward when the protective cover is moved to a position around the distal end portion of the pricking member that protrudes from the used lancet as described above. In other words, the spring S3 functions as a push spring.

As will be easily understood, when the cocking member 212 is moved backward to retract in relation to the other parts in the shown injector assembly 200 by applying a force to the cocking member, the front end portion of the cocking member 212 causes the partition member 534 provided on the rear end portion of the plunger to move backward. Accordingly, the plunger moves backward so that the protrusion 524 of the plunger moves backward beyond the rear end portion 526 of the trigger member 514. When the force apply to the cocking member 212 is removed in this state, the protrusion 524 of the plunger abuts against the back of the rear end portion 526 of the trigger member 514 while applying a forward force, so that with respect to the plunger, a state ready to eject the lancet has been established. This state is the cocking state which resembles a gun being cocked, and therefore the member 212 is conveniently called the cocking member.

As described above, in the state in which charging of the lancet assembly 100 has been completed as shown in Fig. 16, the plunger 204 has moved backward from the standby position shown in Fig. 10 with respect to the injector body 202. As a result, the rear end portion of the plunger moves backward so that the spring S2 is in a relatively expanded state and the spring S3 is in a relatively contracted state.

It is made possible for the user of the lancet assembly to visually recognize the completion of charging by an arrangement in which a marked part of the plunger can be seen through an opening provided as a window in the injector body half when charging is completed as described above. In the shown embodiment, an indicator 538 is provided laterally to the position where the protrusion 524 is disposed. For example, the indicator is colored by giving a conspicuous color (such as green), and providing an opening 540 is provided in the wall of the body half at a position corresponding to the side position of the indicator 538 in the state in which charging is completed (refer to Fig. 9). With this arrangement, the color can be seen through the opening 540 when charging is completed, thereby allowing it to confirm that charging has been completed.

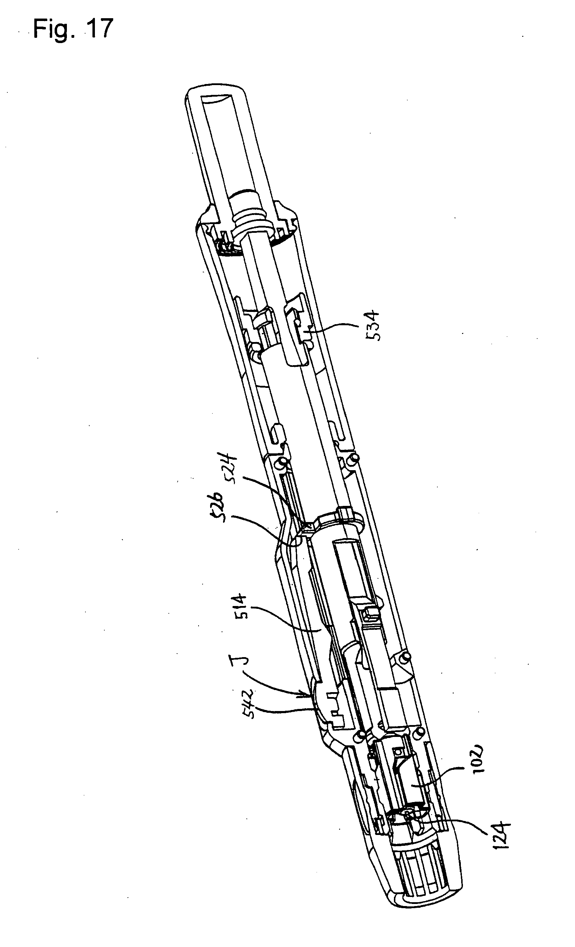

When charging is completed, the lancet cap is removed next. This is carried out by turning the lancet cap around the ejecting direction with respect to the injector assembly that has the lancet held by the plunger as shown in Fig. 16, namely twisting the lancet cap as indicated by the arrow G in Fig. 16 so as to break the weakened portion 108 of the lancet and then pulling off lancet cap 106 forward. The state in which the lancet cap has been removed is shown in Fig. 17.

In order to prevent the lancet body from turning simultaneously when the lancet cap is twisted as described above, the protrusion 131 is provided on the rear end portion of the lancet body, and at least a part of the protrusion is fitted in a recess formed in the rear portion of the inside surface(s) of the plunger. The protrusion may have any shape as long as it is not axially symmetrical with respect to the axis of the pricking member. For example, the protrusion may have such a shape that protrudes in a line form, for example in the form of alphabet letter "I", or "X" (namely cross) on the rear end surface. In the embodiment shown in Fig. 3, the protrusion 131 has the X-letter shape and the plunger has a recess 242 in which a part of the protrusion fits.

Then, the predetermined portion to be pricked, for example, a finger tip is applied to the opening 214 of the injector assembly. In this state, a portion of the front end portion 542 of the trigger member 514 that is exposed as a push button on the upper side surface of the injector body is depressed downward as indicated by the arrow J. This causes the rear end portion 526 of the trigger member 514 to move upward so that the abutting relationship between the rear end portion 526 and the protrusion 524 of the plunger is broken, and the compressed spring S1 is released to expand instantaneously. Accordingly, the lancet body 104 having the exposed distal end portion 124 of the pricking member 105 moves forward in an instant so as to protrude through the opening 214 of the injector assembly and prick the predetermined portion. Then, the lancet body moves reversely, namely backward and the plunger returns to the position which is the same as that of standby state.

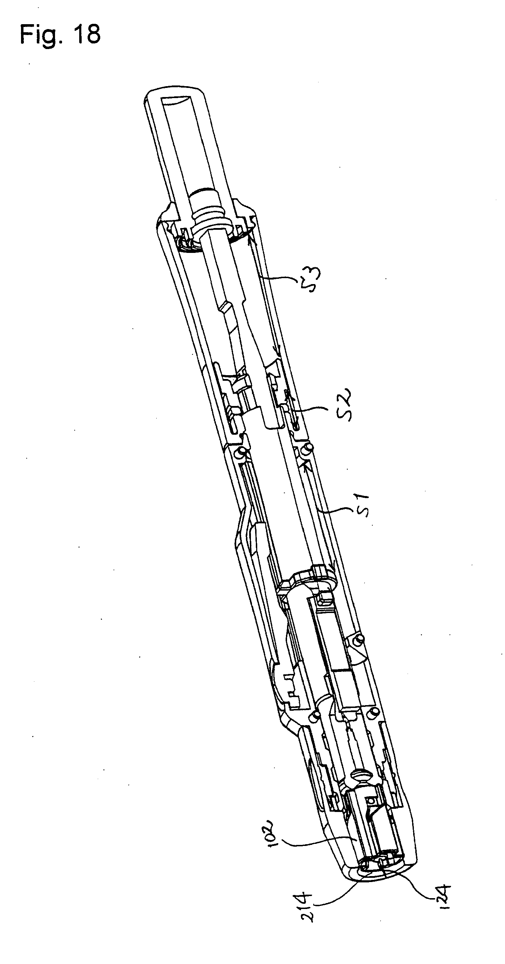

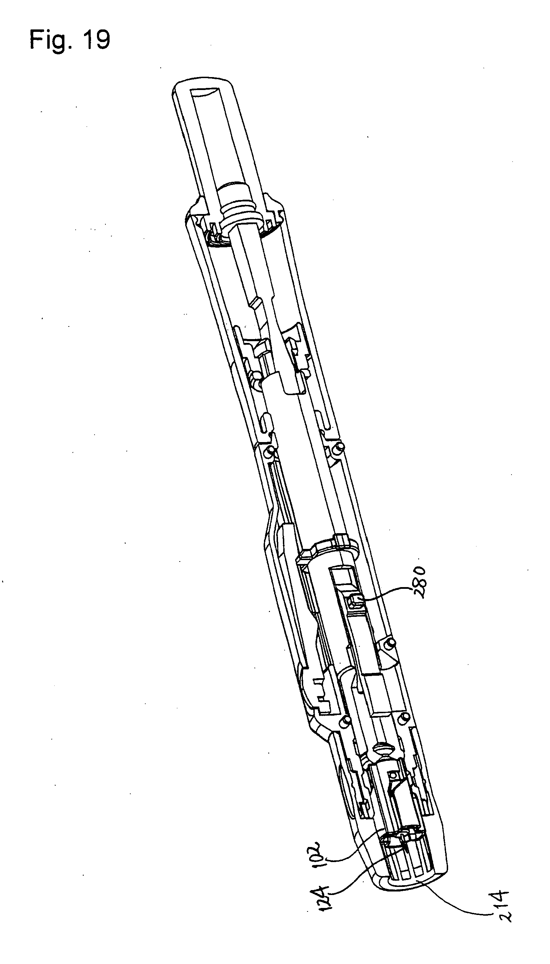

Fig. 18 shows the state in which the distal end portion 124 of the pricking member has moved to the foremost position (namely the state of pricking the predetermined portion), and Fig. 19 shows the state in which the plunger has fully retracted thereafter. This state is followed by a step of removing the lancet that has been used from the injector assembly for discarding.

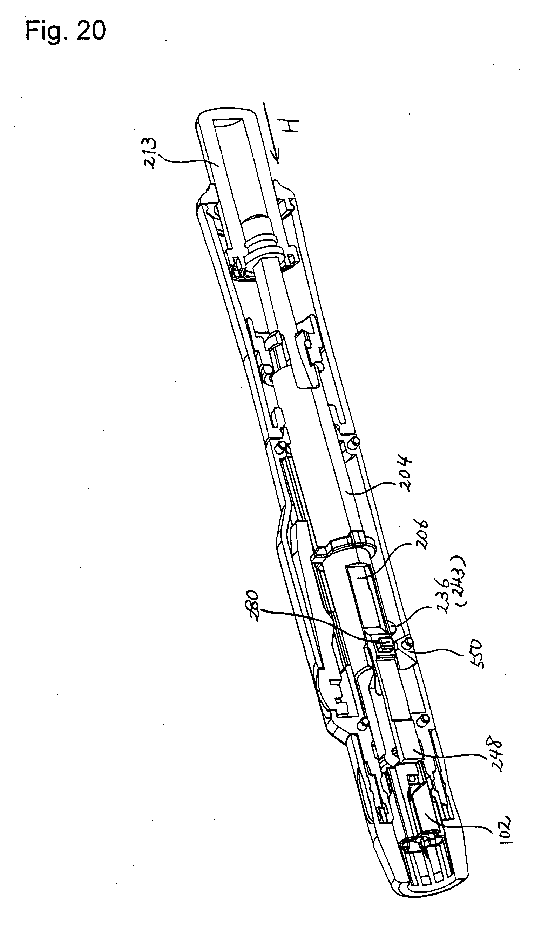

From the state shown in Fig. 19, the push bar 213 is pressed forward first so as to move the pusher 206 forward as indicated by arrow H in Fig. 20. The distal end portions of the arms 246 and 248 of the front portion 250 of the pusher 206 abut against the rear end portion 150 of the protective cover 102 (refer to Fig. 4). In order to make it easier to achieve this abutment, it is preferable that the protective cover 102 has a protrusion(s) 152 that protrudes transversely on the rear end portion thereof. Such abutment state is shown in Fig. 20. To assist the pusher 208 in this movement, the pusher 206 has a protrusion 280 on a side of the arm, and such protrusion 280 can move back and forth in a channel formed in on the inner wall of the lancet body.

When the push bar 213 is pressed forward further from the state shown in Fig. 20 so as to move the pusher 206 further forward, the protective cover 102 is caused to move forward by the arms 246 and 248, so that the rod-like portion 244 of the pusher moves while sliding in the space 260 formed in the rear portion 226 of the plunger 204. Then, the recess 247 formed in the base of the pusher arms and the sloped surface 249 that is located behind the recess touch the sloped surface 235 of the cantilever 232 of the plunger. As a result, the cantilever 232 deflects downward as shown in Fig. 20, and the front end portion of the cantilever protrudes from the bottom of the plunger. It is preferable to provide the protrusion 243 shown in the drawing similarly to the protrusion 237 also on the lower side of the front end portion of the cantilever.

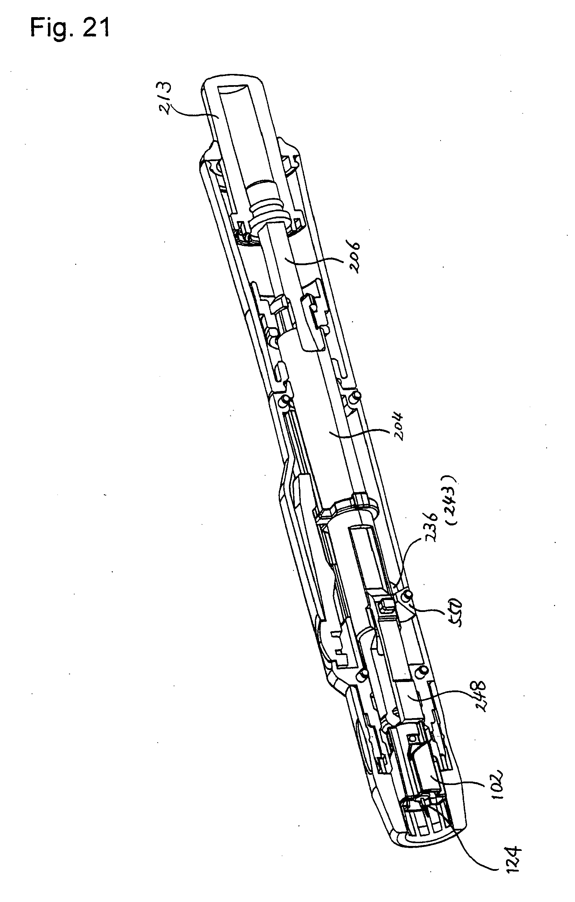

Thus deflected front end 236 or protrusion 243 abuts against the injector body, specifically the inner wall of the main body of the injector body, so as to function as a brake, in order to prevent the forward movement of the plunger even when a force is acting on the plunger 204 to push it forward. In a preferred embodiment, a protrusion 550 is provided as a stopper on the inner wall of the injector body as shown in the drawing. In this case, the deflected front end 236 or protrusion 243 can easily abut against the protrusion 550, so that thereafter the plunger is not capable of moving forward any more. Fig. 21 shows the state of the front end 236 in such contact with the stopper 550.

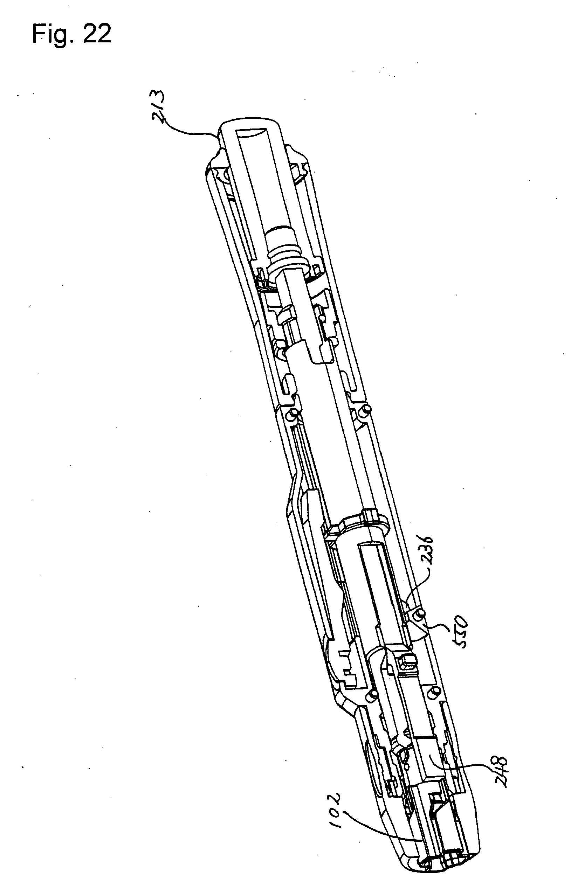

When the forward movement of the plunger 204 is prevented as described above, the lancet body 104 is prevented from moving forward. On the other hand, when the push bar 213 is pressed further forward, a forward-directed force acts on the rear end of the protective cover 102. As a result, only the protective cover 102 moves fully forward to a position around the protruding distal end portion of the pricking member, so that the tip of the distal end portion 126 of the pricking member is located at a position which is sufficiently behind the front end portion of the protective cover 128, as the state shown in Fig. 22. In this state, the relationship between the protective cover and the lancet body 104 is substantially the same as that shown in Fig. 6 or Fig. 7. In this state, the protective cover 102 has been moved fully forward, and the protrusions 122 of the lancet body is fitted into the openings 124 formed in the rear end portion of the protective cover. Therefore, the protective cover 102 is unable to make substantial movement forward nor backward from this state. As will be understood from the drawing, the outermost portions 502 and 504 of the distal end portions of the plunger arms 228 and 230 are adjacent to the injector body, specifically the inner wall of the main body of the injector body, and therefore the arms 228 and 230 are not capable of splaying outwardly from the state shown in the drawing.

Then, as the push bar 213 is pressed further forward, the pusher 206 moves forward with respect to the plunger 204 while deflecting the cantilever 232. Then, the protrusion 236 of the front end portion of the cantilever 232 fits into the opening 262 of the pusher, so that the cantilever 232 restores its original state (recovers from the deflected state). At this stage, the front end portion of the cantilever loses its function of the stopper, and therefore the forward movement of the plunger 204 becomes possible. Therefore, when the pusher 206 causes the plunger to move forward and the front end portions 264 and 266 of the plunger arms reach the recesses 560 and 562 formed in the inner wall of the injector body, the arms 228 and 230 can splay outward. At this time, since the pusher 206 is already pressing the rear end of the protective cover 102 forward, the lancet body receives a forward-directed force and therefore the arms 228 and 230 splay out. As a result, the lancet body, particularly the protrusion 118 thereof is released from the recesses 239 and 241 of the arms and is then pushed forward.

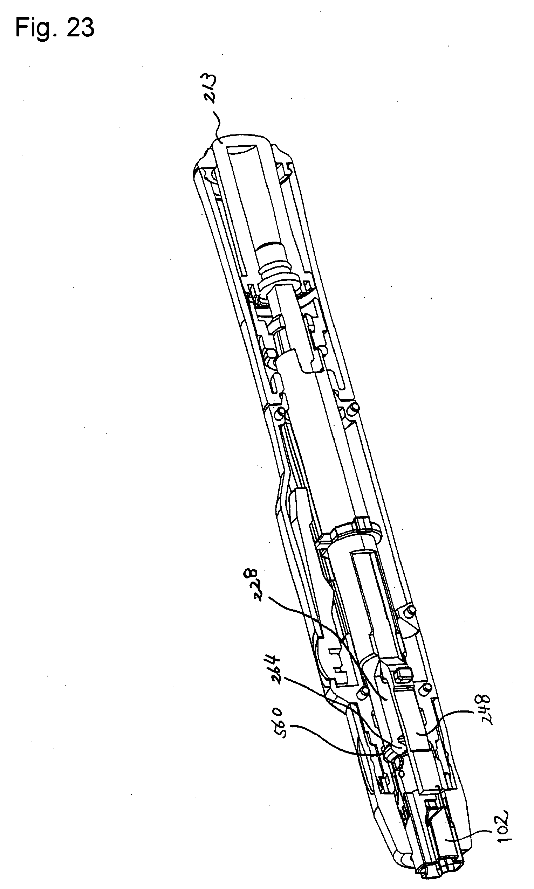

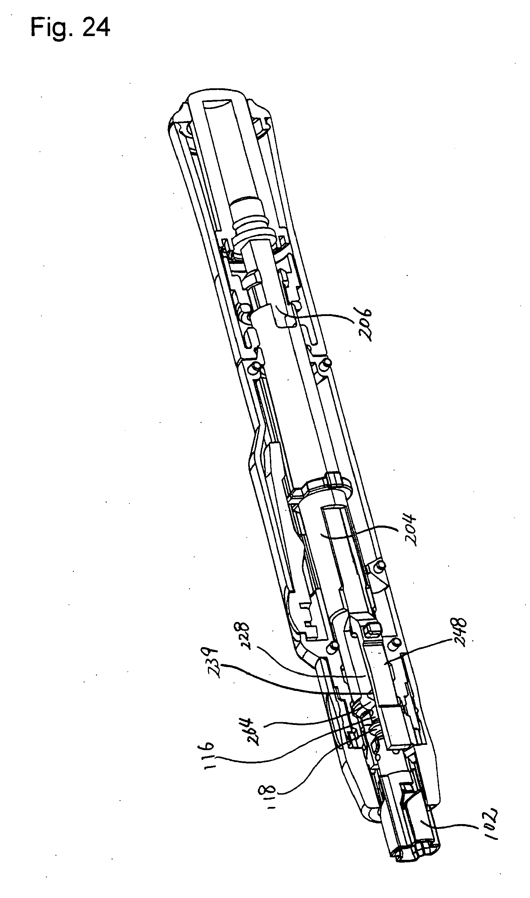

Fig. 23 shows the state in which the front end portions 264 and 266 of the plunger arms reach the recesses 560 and 562 formed in the inner wall of the injector body, with the arms 228 and 230 splaying outward, and Fig. 24 shows the state in which the rear end portion 116 of the lancet body has been released thereafter from the recesses the 239 and 241 of the arms.

As will be easily understood, the pusher may have two opposing arms as described above, preferably arms having U-letter shape as a whole (as shown in the drawing). In other embodiment, more than two arms may be provided as long as the rear end portion of the protective cap can be properly pushed. Thus the pusher has at least two arms.

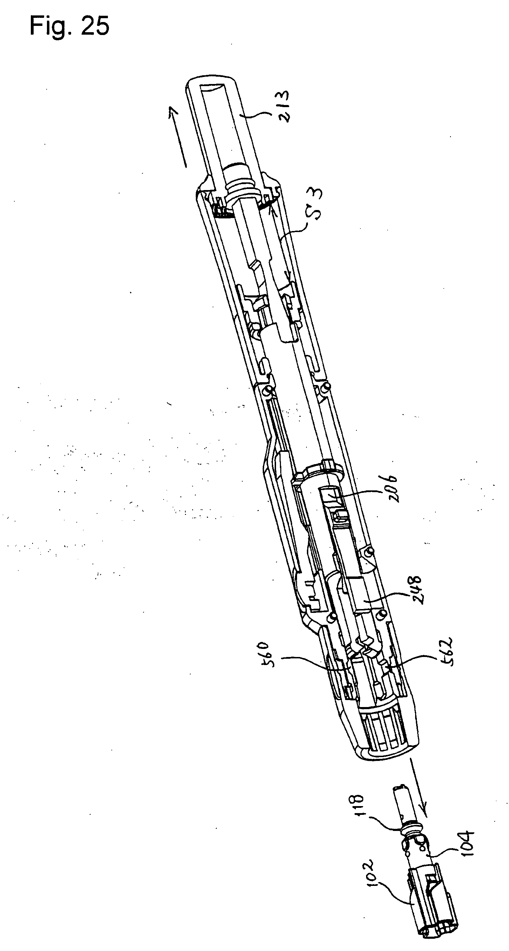

In the state shown in Fig. 22, since the stopper 550 is functioning, the plunger 204 cannot move forward, and therefore the protective cover 102 of which rear end portion is in contact with the pusher arms 246 and 248 is also unable to move forward. In case the position of the recess 262 of the pusher 204 (accordingly the distance between the recess 247 and the recess 262) is so designed that only the pusher 206 can move forward a little in the above mentioned state, the arms 246 and 248 of the pusher 206 deflect a little before the front end portions of the plunger arms 228 and 230 release the protrusion 118 of the lancet body. Immediately thereafter, when the front end portions 264 and 266 of the plunger arms reach the recesses 560 and 562, the pusher arms 246 and 248 move to restore the original shape substantially at the same time as the arms 228 and 230 splay outwardly so that a forward moving forces is instantaneously applied to the lancet body of which protrusion 118 has been released. As a result, the lancet of which pricking member is surrounded by the protective cover shoots out of the opening 214 of the injector assembly, as shown in Fig. 25. The push bar 213 is shown in the state in which the force acting thereon has been released, and has restored its original state (namely, in the standby state) due to the action of the spring S3.

In the state in which the lancet has shot out or has been released as described above, the protective cover is disposed around the pricking member, and the tip of the end portion of the pricking member is located at a position sufficiently deep from the front end surface of the protective cover 102. As a result, even when handling the lancet that has been used, the possibility of touching the tip of the distal end portion of the pricking member that has pricked is greatly reduced.

In this way, the operation of removing the lancet from the injector assembly after pricking can be easily done simply by pressing the push bar 213 forward so as to dispose the protective cover to the tip of the distal end portion of the pricking member as described above, without directly handling the lancet, when the pricking device that employs the lancet assembly and the injector assembly of the present invention is used.

It is noted that in the description given above, charging of the lancet assembly is completed by pressing the lancet assembly backward. In other embodiment, charging may be completed also in such a procedure as, after having the rear end portion of the lancet body held by the opposing arms of the plunger, the cocking member 213 is pulled backward with respect to the lancet body 220 so as to dispose the protrusion 524 of the plunger at a position behind the rear end portion 526 of the trigger member 514.

As will be easily understood from the foregoing description, when pricking operation is carried out using the pricking device that employs the lancet assembly and the injector assembly of the present invention, the process from charging of the lancet assembly in the injector assembly up to discharging of the used lancet from the injector assembly can be carried out substantially in the following five steps:

### Step 1 (Charging of Lancet Assembly)

The lancet assembly 100 is inserted through the opening 214 of the injector assembly 200 and is pressed inward (the step of changing from the state as shown in Fig. 10 to the state as shown in Fig. 16). This causes the arms 228 and 230 of the plunger 204 to hold the rear end portion 116 of the lancet body, and also move backward while gradually pushing up the rear portion of the trigger member 514 against the force exerted by the spring 520. When the protrusion 524 of the plunger passes right below the rear end portion 526 of the trigger member 514, the spring 520 expands so as to cause the rear end portion 526 to move downward. As a result, the protrusion 524 becomes in the state in which it abuts against the rear end portion 526 of the trigger member 514, thereby completing the charging (or loading) of the lancet assembly to be ready for pricking operation. It is noted that upon such abutment, the rear end portion of the trigger member hits the upper arm of the plunger so as to generate a light click sound or a tactile impact, allowing the operator readily to know the completion of charging.

### Step 2 (Removing of Lancet Cap)

The lancet cap 106 that protrudes from the front end of the injector assembly is twisted to break the weakened portion 108. Then, the lancet cap 106 is removed by pulling out, so as to cause the tip of the distal end portion of the pricking member to protrude from the front end portion of the lancet body, to reach the state as shown in Fig. 17.

### Step 3 (Ejecting of Lancet)

The push button 542 is depressed downward as indicated by arrow J in Fig. 17 so as to launch the lancet 101, in which the tip of the distal end portion 214 of the pricking member is protruding, toward the predetermined portion. The lancet 101 that has been launched pricks the predetermined portion in which is applied to the opening 214 (the state shown in Fig. 18), and then retracts (the state shown in Fig. 19) .

### Step 4 (Discharging of Used Lancet)

Then, the push bar 213 is pressed forward (the state shown in Fig. 20), so as to move the protective cover 102 forward by means of the pusher 206 (the state shown in Fig.21) and dispose the protective cover 102 at a position around the protruding distal end portion 124 of the pricking member (the state shown in Fig. 22). Then, the pusher is pressed further to discharge the lancet 101 through the opening 214 of the injector assembly (the states shown in Fig. 24 and Fig. 25). The lancet that has been discharged can be properly disposed of.

When the pricking operation is carried out using the lancet disclosed in U.S. Patent No. 5,385,571, the following nine steps are required from charging of the lancet assembly in the injector assembly to discharging of the used lancet from the injector assembly:

(1) The injector cap is removed from the injector.
(2) The lancet is fitted into the injector.
(3) The lancet cap is removed from the lancet that has been fitted into.
(4) The injector cap that has been removed as described above is put on the injector.
(5) The injector is cocked and the lancet is charged.
(6) The trigger button is pressed to launch the lancet.
(7) The injector cap is removed.
(8) The lancet cap is put on the distal end portion of the pricking member that is exposed so as to shield the distal end portion.
(9) The lancet is removed from the injector so as to dispose of the used lancet.

As will be easily understood by the comparison, when the pricking operation is carried out using the pricking device that employs the lancet assembly and the injector assembly of the present invention, the number of steps for the pricking is greatly reduced and the pricking operation is made simpler.

### Industrial Applicability

As will be apparent from the foregoing description, the lancet assembly and the injector assembly of the present invention provide the pricking device that enables it to safely take an amount of blood sample.

### Cross-Reference to Related Application

This application claims a priority based on Japanese Patent Application No. 2005-231649 (filing date: August 10, 2005, Title of the Invention: PRICKING DEVICE, AND LANCET ASSEMBLY AND INJECTOR ASSEMBLY THAT CONSTITUTE THE SAME), the disclosure of which is incorporated by reference herein.

## Claims

1. A pricking device comprising:
(a) a lancet assembly comprising a lancet and a protective cover; and
(b) an injector assembly that has an injector body, and a plunger and a pusher which are disposed in the injector body and which ejects the lancet,
**characterized in that**
(1) the lancet comprises a lancet body, a lancet cap and a pricking member, the pricking member is disposed in the lancet body and the lancet cap while straddling those members, and a distal end portion of the pricking member is enclosed by the lancet cap;
(2) the protective cover is disposed around the lancet body, and it is able to move to a position, after pricking, around the distal end portion of the pricking member that protrudes forward from the lancet body;
(3) the plunger holds a rear end portion of the lancet body and ejects the lancet body so that the protruding distal end portion of the pricking member pricks a predetermined portion; and
(4) the pusher pushes, after pricking, the protective cover that is disposed around the lancet body so as to move it forward, and thereby the protective cover is located around the distal end portion of the pricking member that protrudes forward from the lancet body.

2. The pricking device according to claim 1, **characterized in that** forward movement of the plunger is prevented when the pusher is moved forward relative to the plunger in order that the pusher pushes forward the protective cover located around the lancet body.

3. The pricking device according to claim 1 or 2, **characterized in that** the plunger comprises a rear portion which is in the form of a cylinder as a whole, and a front portion comprising at least two arms which extend forward from a front end of said rear portion.

4. The pricking device according to any one of claims 1 to 3, **characterized in that** the pusher comprises a rod-like portion and a front portion comprising at least two arms which extend forward from a front end of said rod-like portion,
front end portions of the arms are able to push a rear end of the protective cover so as to move the protective cover forward, and
the rod-like portion is able to move in a space within the rear portion of the plunger while a portion of the rod-like portion is protruding from each end of the rear portion of the plunger.

5. The pricking device according to any one of claims 1- to 4, **characterized in that** at least one arm of the plunger comprises a cantilever which is able to elastically deform so as to contact with an inner wall of the injector body,
the pusher deforms the cantilever elastically so that a front end of the cantilever moves toward the inner wall of the injector body when the pusher is moved forward in an ejecting direction and relative to the plunger so as to push the protective cover forward.

6. A lancet assembly comprising a lancet and a protective cover, **characterized in that**
(1) the lancet comprises a lancet body, a lancet cap and a pricking member, the pricking member is disposed in the lancet body and the lancet cap while straddling those members, and a distal end portion of the pricking member is enclosed by the lancet cap; and
(2) the protective cover is disposed around the lancet body, and it is able to move to a position, after pricking, around the distal end portion of the pricking member that protrudes forward from the lancet body.

7. The lancet assembly according to claim 6, **characterized in that** the lancet body and the lancet cap are connected integrally through a weakened portion located between them, and the weakened portion is broken by twisting the lancet body and the lancet cap around their axis in relatively opposing directions.

8. The lancet assembly according to claim 6 or 7, **characterized in that** the lancet body comprises, around its rear portion and preferably around a whole periphery of its rear portion, a protrusion which is grasped by a chuck element of the plunger of an injector assembly which plunger ejects the lancet.

9. The lancet assembly according to claim 8, **characterized in that** the protrusion is in the form of a rim.

10. The lancet assembly according to any one of claims 6 to 9, **characterized in that** the protrusion of the lancet body comprises a sloped or tapered surface which extends from a top of the protrusion to a lancet body surface, and a chuck element of an injector assembly comprises a sloped or tapered surface in a front end portion of the chuck element which latter sloped or tapered surface splay out forward complementarily with the former sloped or tapered surface, and
upon inserting the lancet into the injector assembly, when the protrusion abuts against the chuck element of the injector assembly, and then the lancet is urged to move further backward by application of a force, the sloped or tapered surfaces make the chuck element splay out toward an inner wall of the injector assembly, so that the lancet is moved backward, and then the protrusion of the lancet body is fitted into a recess which is located behind the sloped or tapered surface.

11. The lancet assembly according to any one of claims 6 to 10, **characterized in that** a rear end surface of the lancet body comprises a protrusion, and a pedestal which is provided at a deepest portion of a chuck element of a lancet ejecting plunger of an injector assembly and which receives the rear end surface of the lancet body has a recess which is complementary with at least a portion of said protrusion.

12. The lancet assembly according to claim 11, **characterized in that** the protrusion is a cross-shaped protrusion.

13. The lancet assembly according to any one of claims 6 to 12, **characterized in that** the lancet cap comprises a protrusion at its rear end portion, and a front end portion of the protective cover abuts against the protrusion of the lancet cap when the protective cover is located around the lancet body.

14. The lancet assembly according to any one of claims 6 to 13, **characterized in that** the lancet body comprises a protrusion for preventing its return which protrudes from a side of the lancet body,
the protective cover has, in its side, an opening into which the protrusion for preventing return is fitted, and
upon the movement of the protective cover so as to locate around the distal end portion of the pricking member which portion protrudes forward from the lancet body, the protrusion is fitted into the opening after a wall forming the protective cover goes over the protrusion.

15. The lancet assembly according to any one of claims 6 to 14, **characterized in that** the protective cover comprises an abutment element against which a front end of an arm of the pusher of the injector assembly abuts.

16. The lancet assembly according to any one of claims 6 to 15, **characterized in that** an outer profile of a cross section of the protective cover which section is perpendicular to an ejecting direction is complementary with a profile of a cross section of an inner wall of a front portion of the injector body which is section is perpendicular to the ejecting direction.

17. An injector assembly comprising an injector body and a plunger and a pusher which are disposed in the injector body and which ejects a lancet that has a protective cover,
**characterized in that**
(1) the plunger holds a rear end portion of the lancet body and ejects the lancet body so that a protruding distal end portion of the pricking member pricks a predetermined portion; and
(2) the pusher pushes, after pricking, the protective cover that is disposed around the lancet body so as to move it forward, and thereby the protective cover is located around the distal-end portion of the pricking member that protrudes forward from the lancet body.

18. The injector assembly according to claim 17, **characterized in that** the injector body comprises a cap assembly, a pair of body halves and a cocking member.

19. The injector assembly according to claim 17 or 18, **characterized in that** the cap assembly comprises an opening against which a predetermined portion to be pricked is forced, and a distance between the opening and front ends of the body halves is able to be changed.
